# EUROPEAN PATENT APPLICATION

(11) **EP 4 066 798 A1**
(43) Date of publication of application: **05.10.2022**
(21) Application number: 20892283.1
(22) Date of filing: 23.11.2020
(51) Int. Cl.: A61F 11/00, H04R 25/00, A61B 5/12, A61H 15/00, A61H 9/00

(54) **MASSAGE DEVICE PROVIDING TINNITUS TREATMENT PROGRAM AND CONTROL METHOD THEREFOR**

(30) Priority: 26.11.2019 KR 20190153743
(71) Applicant: Bodyfriend Co., Ltd., Seoul 06302 (KR)
(72) Inventor: CHO, Younghoon, Seoul 06302 (KR)
(74) Representative: Frenkel, Matthias Alexander
(86) International application number: PCT/KR2020/016579
(87) International publication number: WO 2021/107531

(57) **Abstract**

Disclosed are a massage device providing a tinnitus treatment program and a control method therefor. According to an embodiment of the present disclosure, disclosed is a method by which a massage device provides a tinnitus treatment program. The method by which the massage device provides the tinnitus treatment program comprises the steps of: obtaining tinnitus information about a user; adjusting the magnitude of at least some of a plurality of frequency components included in a raw sound source and then flattening the raw sound source, so as to generate a first processed sound source; reducing the magnitude of at least some of a plurality of frequency components included in the first processed sound source on the basis of the obtained tinnitus information, so as to generate a tinnitus treatment sound source; and providing a tinnitus treatment program in which the generated tinnitus treatment sound source and tinnitus treatment massage are combined.

## Description

### [Technical Field]

The present disclosure relates to a method by which a massage device provides a tinnitus treatment program, and a massage device providing the tinnitus treatment program.

### [Background Art]

According to the National Health and Nutrition Examination Survey in 2017, tinnitus is a symptom experienced by about 20% of adults in Korea. In addition, about 5% of the total population suffers from tinnitus that is "annoying or irritating or makes it difficult to sleep."

Tinnitus is a ringing phenomenon in the ears, and means a state felt in the brain without external sound source stimulation. For tinnitus perceived in the cerebrum, the limbic system feels unpleasant emotions related to tinnitus and feels insomnia, anxiety, and fear according to the response of the autonomic nervous system. These reactions again act as aggravating factors for tinnitus, and make tinnitus worse.

In the treatment of tinnitus, methods such as sound therapy, pharmacotherapy, percutaneous magnetic field stimulation treatment, tinnitus retraining treatment, and the like have been tried. However, due to the complex mechanism of tinnitus, tinnitus takes a long period of time to treat, does not have a high success rate, and recurs frequently.

Among the sound therapy methods, sound therapy with more than shielding sound can interfere with the habituation of tinnitus and can be effective immediately after the use of the sound, but has a disadvantage that tinnitus will start again when the treatment is stopped.

In tinnitus retraining treatment, in which sound therapy with more than shielding sound and counseling therapy are combined, the negative factors that the patient feels from tinnitus can be removed, and adaptation of the central nervous system can be induced, and thus a long-term therapeutic effect can be expected. However, the treatment period to acquire the effect of tinnitus retraining treatment is long, more than several months, and there is a feeling of discomfort in having to visit the hospital periodically during that period.

Any type of sound therapy described above uses sound such as white noise, broadband noise, or the like. However, because such sound causes discomfort and boredom in listening for a long period of time, patient compliance is low, and thus the success rate of treatment is low.

Since the sound therapy uses white noise or broadband noise, discomfort and boredom are caused. Therefore, there are many cases of tinnitus patients giving up in the middle of treatment, and thus the success rate is low.

For tinnitus retraining treatment, it is necessary for patients to visit the hospital regularly and receive counseling therapy, which causes discomfort.

Pharmacotherapy also requires taking drugs for a long period of time, and there is a limit in that the purpose is to relieve tinnitus symptoms rather than cure tinnitus.

Various treatment methods, including intracranial magnetic field stimulation and the like, which have been recently implemented by various research groups, have unclear effects and remain at the research stage.

Therapy of frequency-modified music suitable for the characteristics of the patient has less discomfort and boredom than the conventional sound therapy using white noise or broadband noise, but requires listening to music for a long period of time, for example, at least 30 minutes, due to the characteristics of sound therapy, and thus boredom is also present.

Further, the actual aspect of tinnitus has a high-frequency aspect, and an intensity of a general sound source decreases as the pitch increases (-12,000 Hz), and thus there is a need for a method of treating tinnitus for patients with tinnitus in the high-pitched sound range.

A technique for treating tinnitus using music is disclosed in Korean Registered Patent No. 10-1057661.

### [Disclosure]

### [Technical Problem]

The present disclosure has been derived in response to the above background art, and is directed to providing a method by which a massage device provides a tinnitus treatment program, and a massage device providing the tinnitus treatment program.

### [Technical Solution]

One aspect of the present disclosure provides a method by which a massage device provides a tinnitus treatment program, including acquiring tinnitus information of a user; generating a first processed sound source by adjusting a magnitude of at least some frequency components among a plurality of frequency components included in a raw sound source and flattening the raw sound source; generating a tinnitus treatment sound source by reducing a magnitude of at least some frequency components among a plurality of frequency components included in the first processed sound source on the basis of the acquired tinnitus information; and providing a tinnitus treatment program in which the generated tinnitus treatment sound source and a tinnitus treatment massage are combined.

Another aspect of the present disclosure provides a massage device providing a tinnitus treatment program, including a tinnitus information acquisition unit configured to acquire tinnitus information of a user; a first processed sound source generation unit configured to generate a first processed sound source by adjusting a magnitude of at least some frequency components among a plurality of frequency components included in a raw sound source and flattening the raw sound source; a tinnitus treatment sound source generation unit configured to generate a tinnitus treatment sound source by reducing a magnitude of at least some frequency components among a plurality of frequency components included in the first processed sound source on the basis of the acquired tinnitus information; and a tinnitus treatment program providing unit configured to provide a tinnitus treatment program in which the generated tinnitus treatment sound source and a tinnitus treatment massage are combined.

### [Advantageous Effects]

The effects obtainable in the present disclosure are not limited to the above-described effects and other effects that do not described may be clearly understood by those skilled in the art from the following description.

The massage device according to an embodiment of the present disclosure can relieve or treat tinnitus symptoms of the user by providing a tinnitus treatment program in which a tinnitus treatment sound source generated according to the tinnitus information of the user and a tinnitus treatment massage are combined.

Various aspects are now described with reference to the accompanying drawings, wherein like reference numbers are used to refer to like elements throughout. In the following embodiments, for purposes of description, numerous specific details are set forth in order to provide a thorough understanding of one or more aspects. It will be clear, however, that such aspect(s) may be practiced without these specific details. In other examples, well-known structures and devices are shown in block diagram form in order to facilitate describing one or more aspects.

### [Description of Drawings]

FIG. 1 is a view for describing a massage device (1000) according to an embodiment of the present disclosure.
FIG. 2 is a view for describing a main frame according to an embodiment of the present disclosure.
FIG. 3 is a view for describing components of a massage device (1000) according to an embodiment of the present disclosure.
FIG. 4 is a flowchart for describing a method by which a massage device provides a tinnitus treatment program according to an embodiment of the present disclosure.
FIG. 5 is a flowchart for describing a method of acquiring a tinnitus frequency band according to an embodiment of the present disclosure.
FIGS. 6A and 6B are graphs for describing a method of generating a first processed sound source according to an embodiment of the present disclosure.
FIG. 7 is a graph for describing a method of determining a slope of a virtual line according to an embodiment of the present disclosure.
FIG. 8 is a graph for describing a method of generating a tinnitus treatment sound source according to an embodiment of the present disclosure.

### [Best mode of the Invention]

Disclosed is a method by which a massage device provides a tinnitus treatment program, the method including acquiring tinnitus information of a user; generating a first processed sound source by adjusting a magnitude of at least some frequency components among a plurality of frequency components included in a raw sound source and flattening the raw sound source; generating a tinnitus treatment sound source by reducing a magnitude of at least some frequency components among a plurality of frequency components included in the first processed sound source on the basis of the acquired tinnitus information; and providing a tinnitus treatment program in which the generated tinnitus treatment sound source and a tinnitus treatment massage are combined.

### [Modes of the Invention]

The objects, features, and advantages of the present disclosure described above will become more apparent from the following embodiments related to the accompanying drawings. The following specific structural and functional descriptions are only for the purpose of describing the embodiments of the present disclosure, and the embodiments of the present disclosure may be embodied in various forms and are not to be construed as limited to the embodiments described in this specification or application.

While the present disclosure is susceptible to various modifications and alternative forms, specific embodiments thereof are shown by way of example in the accompanying drawings and will be described in this specification or application in detail. However, it should be understood that there is no intent to limit the present disclosure to the particular forms disclosed, and on the contrary, the present disclosure is to cover all modifications, equivalents, and alternatives falling within the spirit and scope of the present disclosure.

It should be understood that, although the terms "first," "second," and the like may be used herein to describe various elements, the elements are not limited by the terms. The terms are only used to distinguish one element from another element. For example, a first element could be called a second element, and, similarly, a second element could be called a first element, without departing from the scope of the present disclosure.

It should be understood that when an element is referred to as being "connected" or "coupled" to another element, the element may be directly connected or coupled to another element or intervening elements may be present. In contrast, when an element is referred to as being "directly connected" or "directly coupled" to another element, there are no intervening elements present. Other words used to describe the relationship between elements should be interpreted in a like fashion (i.e., "between" versus "directly between," "adjacent" versus "directly adjacent," and the like.).

The terminology used herein is for the purpose of describing particular embodiments only and is not intended to limit the present disclosure. As used herein, the singular forms "a" and "an" are intended to also include the plural forms, unless the context clearly indicates otherwise. It should be further understood that the terms "comprise," "comprising," "include," and/or "including," when used herein, specify the presence of stated features, integers, steps, operations, elements, parts, or combinations thereof, but do not preclude the presence or addition of one or more other features, integers, steps, operations, elements, parts, or combinations thereof.

Unless otherwise defined, all terms including technical and scientific terms used herein have the same meaning as commonly understood by one of ordinary skill in the art to which the present disclosure belongs. It should be further understood that terms, such as those defined in commonly used dictionaries, should be interpreted as having a meaning that is consistent with their meaning in the context of the relevant art and are not to be interpreted in an idealized or overly formal sense unless expressly so defined herein.

In this specification, an actuator refers to a component that can provide a driving force. For example, the actuator may include a motor, a linear motor, an electronic motor, a direct current (DC) motor, an alternating current (AC) motor, a linear actuator, an electric actuator, and the like, but the present disclosure is not limited thereto.

In this specification, a spiral rod refers to a straight member having a spiral groove and may be implemented with a metal material. For example, the spiral rod may include a cylindrical rod having a spiral groove in a surface thereof. Further, the spiral rod may include a metal lead screw.

In this specification, binaural beats may refer to a specific type of audio information that can be used for adjusting brain waves.

In this specification, according to an embodiment, a massage device may be referred to as a massage device including a body massage unit and a leg massage unit. Further, according to another embodiment, a body massage unit 100 and a leg massage unit 300 may be present as separate devices (e.g., a body massage device and a leg massage device) and a massage device may be referred to as a body massage device or a leg massage device.

Hereinafter, embodiments of the present disclosure will be described in more detail with reference to the accompanying drawings.

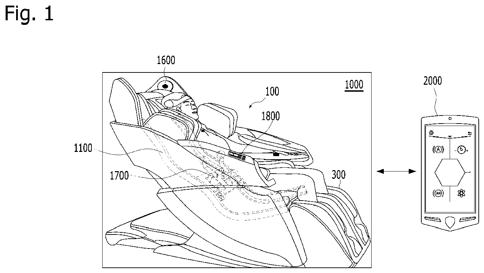

FIG. 1 is a view for describing a massage device 1000 according to an embodiment of the present disclosure.

The massage device 1000 according to the embodiment of the present disclosure may include a body massage unit 100 that forms a region for accommodating at least a part of a user's body and massages the user's body, and a leg massage unit 300 that massages the user's legs.

The body massage unit 100 may provide a massage to at least a part of the user's body. The body massage unit 100 may include a massage module 1700 that provides a massage function to at least a part of the user's body, an audio output unit 1600 that provides an arbitrary type of audio output to the user, a main frame 1100 that forms a framework of the body massage unit 100, and a user input unit 1800 that receives an arbitrary type of input from the user.

The above-described components included in the body massage unit 100 are merely exemplary embodiments, and the body massage unit 100 may include various components in addition to the above-described components.

Further, the shape and structure of the massage device 1000 illustrated in FIG. 1 are merely exemplary, and various types of massage device 1000 may also be included within the scope of the present disclosure unless departing from the scope of rights defined by the claims of the present disclosure.

The body massage unit 100 may form an arbitrary type of space for accommodating the user. The body massage unit 100 may include a space having a shape corresponding to the shape of the user's body. For example, as illustrated in FIG. 1, the body massage unit 100 may be implemented in a sitting type that can accommodate the user's whole body or a part of the body.

A part of the body massage unit 100, which is in contact with the ground, may include an arbitrary material for increasing a frictional force or an arbitrary member (e.g., a non-slip pad or the like) for increasing a frictional force, and may include wheels for enhancing the mobility of the massage device 1000.

At least a part of the body massage unit 100 may slide. For example, when the body massage unit 100 starts a massage, at least a part of the body massage unit 100 may slide forward. Further, the body massage unit 100 may be inclined rearward. As a result, the body massage unit 100 may provide a massage while being inclined rearward.

According to an embodiment of the present disclosure, the massage device 1000 may include at least one air cell (not illustrated). The air cell may be positioned in a part corresponding to the user's shoulder, a part corresponding to the user's pelvis, an arm massage unit, the leg massage unit 300, or the like, but the present disclosure is not limited thereto, and the air cells may be disposed in various parts of the massage device 1000.

The massage device 1000 may include an air supply unit, and the air supply unit may inflate the air cell by supplying air to the air cell. The air supply unit may be positioned inside the body massage unit 100 or may be positioned in the leg massage unit 300. Further, the air supply unit may be positioned outside the massage device 1000.

The leg massage unit 300 may provide a leg massage to the user. For example, the leg massage unit 300 may include a calf massage unit for massaging the user's calf and/or a foot massage unit for massaging the user's feet.

The leg massage unit 300 may be adjustable in length according to the characteristics of the user's body. For example, when a tall user uses the massage device 1000, the length of the leg massage unit 300 needs to be increased because the user has a long calf. Further, when a short user uses the massage device 1000, the length of the leg massage unit 300 needs to be shortened because the user has a short calf. Accordingly, the leg massage unit 300 may provide a leg massage customized to the user's height.

The massage module 1700 may be provided inside the body massage unit 100 to provide an arbitrary type of mechanical stimulation to the user accommodated in the body massage unit 100. As illustrated in FIG. 1, the massage module 1700 may move along the main frame 1100 provided inside the body massage unit 100.

For example, rack gears may be provided in the main frame 1100 of the body massage unit 100, and the massage module 1700 may provide mechanical stimulation to various parts of the user's body while moving along the rack gears. The massage module 1700 may include a ball massage unit or a roller massage unit, but the present disclosure is not limited thereto.

The main frame 1100 is a component that forms a skeleton of the internal configuration of the body massage unit 100, and may be implemented with a metal material, a plastic material, or the like. For example, the main frame 1100 may be implemented with iron, an alloy, steel, etc., but the present disclosure is not limited thereto, and the main frame 1100 may be implemented with any of various hard materials.

According to an embodiment of the present disclosure, the massage device 1000 may include the audio output unit 1600. The audio output unit 1600 may be provided at any of various positions. For example, the audio output unit 1600 may include a plurality of output units, such as an upper audio output unit disposed on an upper end of a seat unit in contact with the user, a front audio output unit attached to a front end of a left or right arm massage unit of the seat unit, and/or a rear audio output unit attached to a rear end of the left or right arm massage unit, but the present disclosure is not limited thereto. In this case, the audio output unit 1600 may provide a stereophonic sound such as 5.1 channel surround sound, but the present disclosure is not limited thereto.

According to an embodiment of the present disclosure, the user may control the massage device 1000 using a massage device control device 2000. The massage device control device 2000 may be connected to the massage device 1000 through wired and/or wireless communication.

The massage device control device 2000 may include a remote controller, a cellular phone, a personal digital assistant (PDA), etc., but the present disclosure is not limited thereto, and the massage device control device 2000 may include various electronic devices connectable to the massage device 1000 through wired or wireless communication.

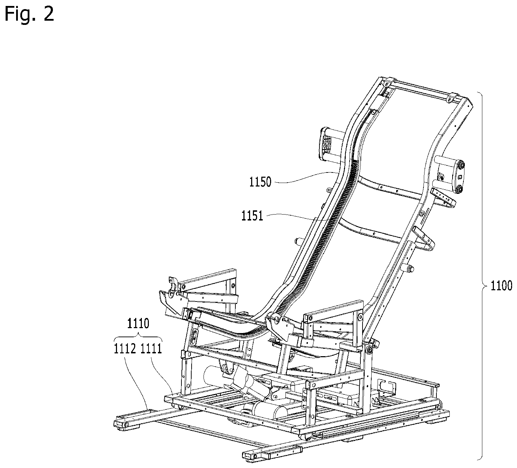

FIG. 2 is a view for describing a main frame according to an embodiment of the present disclosure.

According to an embodiment of the present disclosure, a main frame 1100 may include an upper frame 1150 in which a massage module 1700 is provided and a base frame 1110 that supports the upper frame 1150.

A rack gear 1151 may be provided in at least a part of the upper frame 1150. The rack gear 1151 is a member for guiding the vertical movement of the body massage module 1700, and may include a plurality of valleys and a plurality of ridges.

According to an embodiment of the present disclosure, rack gears 1151 may be provided at both side parts of the upper frame 1150 to face each other, and the body massage module 1700 may move along the rack gears 1151.

For example, the body massage module 1700 may include a gear engaged with the rack gear 1151, and the gear may be rotated by an actuator provided in the body massage module 1700, and thus the body massage module 1700 may move upward or downward.

The rack gears 1151 may be implemented with a metal material or a plastic material. For example, the rack gears 1151 may be implemented with iron, steel, an alloy, reinforced plastic, a melamine resin, a phenol resin, etc., but the present disclosure is not limited thereto.

The upper frame 1150 may be implemented in any of various shapes. For example, the upper frame 1150 may be classified into an S frame, an L frame, an S&L frame, and a double S&L frame according to the shape thereof, but the present disclosure is not limited thereto.

The S frame refers to a frame in which at least a part of the upper frame 1150 has a curved shape like "S." The L frame refers to a frame in which at least a part of the upper frame 1150 has a curved shape like "L," the S&L frame refers to a frame in which a part has both a curved shape like "S" and a curved shape like "L," and the double S&L frame refers to a frame in which a part has a curved shape like an L" and two parts have a curved shape like an "S."

The base frame 1110 refers to a part of the main frame 1100 that supports the upper frame 1150 and is in contact with the ground. The base frame 1110 may include an upper base frame 1111 and a lower base frame 1112.

The upper base frame 1111 may support the upper frame 1150 and the lower base frame 1112 may be in contact with the ground. Further, the upper base frame 1111 may be positioned to be in contact with the lower base frame 1112.

According to an embodiment of the present disclosure, the upper base frame 1111 may move along the lower base frame 1112. For example, the upper base frame 1111 may slide forward or rearward along the lower base frame 1112. In this case, the upper frame 1150 may be connected to the upper base frame 1111 and may move according to the movement of the upper base frame 1111.

For example, when the upper base frame 1111 moves forward, the upper frame 1150 may also move forward, and when the upper base frame 1111 moves rearward, the upper frame 1150 may also move rearward. Accordingly, the sliding movement of the body massage unit 100 may be allowed.

Specifically, in order to allow the movement of the upper base frame 1111, a moving wheel may be provided under the upper base frame 1111. Further, a guide member capable of guiding the moving wheel may be provided above the lower base frame 1112. The moving wheel provided under the upper base frame 1111 may move along the guide member provided above the lower base frame 1112, and thus the forward or rearward movement of the upper base frame 1111 may be allowed.

According to another embodiment of the present disclosure, the massage device 1000 may not provide a sliding function, and in this case, the base frame 1110 may not be divided into upper and lower frames.

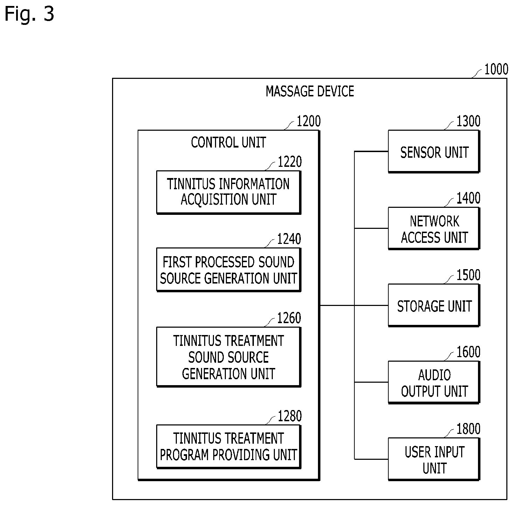

FIG. 3 is a view for describing components of a massage device 1000 according to an embodiment of the present disclosure.

According to an embodiment of the present disclosure, the massage device 1000 may include at least one of a control unit 1200, a sensor unit 1300, a user input unit 1800, an audio output unit 1600, and a network access unit 1400.

The control unit 1200 may control operation of the massage device 1000. The control unit 1200 may be implemented with one processor or may be implemented with a plurality of processors. When the control unit 1200 is implemented with a plurality of processors, at least some of the plurality of processors may be disposed at positions physically spaced apart from each other. Further, the control unit 1200 is not limited thereto and may be implemented in various ways.

According to an embodiment of the present disclosure, the control unit 1200 may control the operation of the massage device 1000. For example, the massage device 1000 may include a plurality of actuators, and the massage device 1000 may control the operation of the massage device 1000 by controlling operation of the plurality of actuators. For example, the massage device 1000 may include at least one of a massage module 1700 movement actuator, at least one actuator included in the massage module, a back angle actuator, a leg angle actuator, a foot massage actuator, a leg length adjustment actuator, and a sliding actuator, and the control unit 1200 may control the operation of the massage device 1000 by controlling the above actuators.

According to an embodiment of the present disclosure, the control unit 1200 may include at least one of a tinnitus information acquisition unit 1220, a first processed sound source generation unit, a tinnitus treatment sound source generation unit 1260, and a tinnitus treatment program providing unit 1280.

The tinnitus information acquisition unit 1220 may acquire tinnitus information of a user.

The tinnitus information acquisition unit 1220 may acquire a tinnitus frequency band of the user in various ways. For example, the tinnitus information acquisition unit 1220 may directly determine the tinnitus frequency band of the user or may acquire the tinnitus frequency band of the user from an external device.

The tinnitus information acquisition unit 1220 may first acquire information about an ear in which tinnitus is heard. For example, the tinnitus information acquisition unit 1220 may provide an interface in which the user can select a left ear, a right ear, or both ears to the user. The user may select the ear in which tinnitus occurs through the provided user interface, and the tinnitus information acquisition unit 1220 may acquire the information about the ear in which tinnitus occurs on the basis of information about the selection by the user. Further, the tinnitus information acquisition unit 1220 may acquire the information about the ear in which tinnitus occurs, which is provided from an external device to the user in advance, but the present disclosure is not limited thereto.

The tinnitus information acquisition unit 1220 may determine a first frequency band in which tinnitus occurs. For example, the tinnitus information acquisition unit 1220 may output a sound source to the left ear, the right ear, or both ears on the basis of the information about the ear in which tinnitus occurs, and may provide an interface in which the user can select a frequency band in which tinnitus occurs together with the sound source. Specifically, the tinnitus information acquisition unit 1220 may output a sound source whose frequency is gradually increased from a low frequency band to a high frequency band (e.g., a sound source whose frequency is gradually increased from 250 Hz to 8,000 Hz for 1 minute is output), provide the interface in which the user can select a frequency band in which tinnitus occurs to the user, and allow the user to directly select the frequency band in which tinnitus occurs.

In this case, the first frequency band may include one frequency. Further, the first frequency band may include a plurality of frequencies, but the present disclosure is not limited thereto.

The tinnitus information acquisition unit 1220 may determine a second frequency band in which tinnitus occurs. For example, the tinnitus information acquisition unit 1220 may output a sound source to the left ear, the right ear, or both ears on the basis of the information about the ear in which tinnitus occurs, and may provide an interface in which the user can select a frequency band in which tinnitus occurs together with the sound source. Specifically, the tinnitus information acquisition unit 1220 may output a sound source whose frequency is gradually reduced from a high frequency band to a low frequency band (e.g., a sound source whose frequency is gradually reduced from 8,000 Hz to 250 Hz for 1 minute is output), provide the interface in which the user can select a frequency band in which tinnitus occurs to the user, and allow the user to directly select the frequency band in which tinnitus occurs.

In this case, the second frequency band may include one frequency. Further, the second frequency band may include a plurality of frequencies, but the present disclosure is not limited thereto.

The tinnitus information acquisition unit 1220 may determine a candidate tinnitus frequency band. For example, the tinnitus information acquisition unit 1220 may determine the candidate tinnitus frequency band on the basis of the determined first and the second frequency bands. Specifically, the tinnitus information acquisition unit 1220 may select at least one frequency band close to the first and second frequency bands from among predetermined frequency bands (e.g., 250 Hz, 500 Hz, 1,000 Hz, 2,000 Hz, 3,000 Hz, 4,000 Hz, 6,000 Hz, and 8,000 Hz) and determine the selected frequency band as the candidate tinnitus frequency band.

In this case, the candidate tinnitus frequency band may include one frequency. Further, the candidate tinnitus frequency band may include a plurality of frequencies, but the present disclosure is not limited thereto.

The tinnitus information acquisition unit 1220 may acquire a tinnitus frequency band. For example, the tinnitus information acquisition unit 1220 may provide the candidate tinnitus frequency band to the user through the interface, and acquire the tinnitus frequency band of the user on the basis of the information about the selection by the user through the interface.

In this case, the tinnitus frequency band may include one frequency. Further, the tinnitus frequency band may include a plurality of frequencies, but the present disclosure is not limited thereto.

According to an embodiment of the present disclosure, the first processed sound source generation unit 1240 may generate a first processed sound source by adjusting the magnitude of at least some frequency components among a plurality of frequency components included in a raw sound source and flattening the raw sound source.

The first processed sound source generation unit 1240 may generate the first processed sound source by adjusting the magnitude of at least some frequency components among the plurality of frequency components included in the raw sound source on the basis of a virtual line having a slope. In this case, the slope of the virtual line may be variously determined.

For example, the first processed sound source generation unit 1240 may determine the slope on the basis of a sympathetic hyperactivity level of the user. For example, the first processed sound source generation unit 1240 may determine the sympathetic hyperactivity level of the user, and determine the slope on the basis of the determined sympathetic hyperactivity level. Specifically, the first processed sound source generation unit 1240 may acquire heart rate variability (HRV) information of the user, determine the sympathetic hyperactivity level of the user by utilizing a ratio (LF/HF ratio) of power in a low frequency band to power in a high frequency band included in the HRV information, and determine the slope on the basis of the determined sympathetic hyperactivity level.

When excessive stimulation is applied to the user whose sympathetic nerve is hyperactive, the user may feel uncomfortable. Accordingly, the first processed sound source generation unit 1240 may generate a sound source with less stimulation by generating the first processed sound source on the basis of a line having a steep slope for a user whose sympathetic nerve is relatively stimulated. According to another embodiment of the present disclosure, a virtual line may be implemented as a curve. Further, the virtual line may be implemented as a stepped line, but the present disclosure is not limited thereto. When the virtual line is implemented as a curve, a slope of the virtual line may be replaced with a slope of the curve. Further, when the virtual line is implemented as a stepped line, a slope of the virtual line may be replaced with a slope of a straight line that connects a start point and an end point of the stepped line, but the present disclosure is not limited thereto.

The tinnitus treatment sound source generation unit 1260 may generate a tinnitus treatment sound source.

According to an embodiment of the present disclosure, the tinnitus treatment sound source generation unit 1260 may generate the tinnitus treatment sound source by reducing the magnitude of at least some frequency components among a plurality of frequency components included in the first processed sound source on the basis of tinnitus information. The tinnitus treatment sound source generation unit 1260 may generate the tinnitus treatment sound source by utilizing a notch filter, but the present disclosure is not limited thereto.

The tinnitus treatment program providing unit 1280 may generate a tinnitus treatment program by combining the tinnitus treatment sound source and a tinnitus treatment massage, and provide the generated tinnitus treatment program to the user.

The tinnitus treatment massage may be determined adaptively according to the user. For example, the tinnitus treatment program providing unit 1280 may determine the tinnitus treatment massage on the basis of the sympathetic hyperactivity level. Specifically, the tinnitus treatment program providing unit 1280 may acquire HRV information, determine the sympathetic hyperactivity level of the user by utilizing a ratio (LF/HF ratio) of power in a low frequency band to power in a high frequency band included in the HRV information, and determine the tinnitus treatment massage on the basis of the sympathetic hyperactivity level.

When excessive stimulation is applied to the user whose sympathetic nerve is hyperactive, the user may feel uncomfortable, and accordingly, the effectiveness of the tinnitus treatment program may be reduced. In order to solve the above problem, the tinnitus treatment program providing unit 1280 may provide a gentle massage (e.g., a massage performed with relatively weak kneading and tapping, a massage performed with a massage module relatively less protruding, and/or a massage performed evenly throughout, rather than intensively massaging one part) to the user whose sympathetic nerve is relatively hyperactive.

According to another embodiment of the present disclosure, the tinnitus treatment massage may be determined based on biometric information of the user. For example, the tinnitus treatment program providing unit 1280 may measure a stress index by analyzing the HRV information of the user. In this case, when it is determined that the stress index is high, the tinnitus treatment program providing unit 1280 may determine a relatively strong upper body massage as the tinnitus treatment massage. Further, when it is determined that the stress index is low, the tinnitus treatment program providing unit 1280 may determine an overall gentle massage (e.g., a massage performed with relatively weak kneading and tapping, a massage performed with a massage module relatively less protruding, and/or a massage performed evenly throughout, rather than intensively massaging one part) as the tinnitus treatment massage.

The massage module movement actuator is an actuator that enables the vertical movement of the massage module 1700, and the massage module 1700 may move along the rack gear by the operation of the massage module 1700 movement actuator.

The back angle actuator is an actuator that adjusts an angle of a part of the massage device 1000, which is in contact with the user's back, and a back angle of the massage device 1000 may be adjusted by the operation of the back angle actuator.

The leg angle actuator is an actuator that adjusts an angle of the leg massage unit 300 of the massage device 1000, and an angle between the leg massage unit 300 and the body massage unit 100 may be adjusted by the operation of the leg angle actuator.

The foot massage actuator is an actuator that operates the foot massage module included in the leg massage unit 300. By utilizing the foot massage actuator, the massage device 1000 may provide a foot massage to the user.

The massage module 1700 may include at least one actuator, and the control unit 1200 may provide various massage operations by operating the at least one actuator. For example, the control unit 1200 may provide a tapping massage, a kneading massage, etc. by operating at least one actuator included in the massage module 1700, but the present disclosure is not limited thereto, and the control unit 1200 may provide various massage operations.

The leg length adjustment actuator is an actuator that adjusts a length of the leg massage unit 300. For example, the control unit 1200 may adjust the length of the leg massage unit 300 according to the user by utilizing the leg length adjustment actuator, and as a result, the user may be provided with a massage suitable for his or her body type.

The sliding actuator enables the sliding operation of the massage device 1000. For example, the upper base frame 1111 may be moved forward or rearward by the operation of the sliding actuator, and as a result, the upper frame connected to the upper base frame 1111 may also be moved forward or rearward.

The sensor unit 1300 may acquire various pieces of information using at least one sensor. For example, the sensors may include a pressure sensor, an infrared sensor, a light-emitting diode (LED) sensor, or the like, but the present disclosure is not limited thereto.

Further, the sensor unit 1300 may include a biometric information acquisition sensor. The biometric information acquisition sensor may acquire fingerprint information, face information, voice information, iris information, weight information, electrocardiogram information, body composition information, and the like, but the present disclosure is not limited thereto, and the biometric information acquisition sensor may include various pieces of biometric information.

According to another embodiment of the present disclosure, the massage device 1000 may detect a contact area and/or a contact position with the user through the sensors. Further, the massage device 1000 may acquire position information of the user's shoulder through the sensor unit 1300. Further, the massage device 1000 may provide a customized massage on the basis of the acquired information. For example, when the massage device 1000 provides a shoulder massage, the massage device 1000 may recognize the position of the user's shoulder on the basis of the information acquired through the sensor unit 1300, and provide a shoulder massage to the user according to a recognition result.

The user input unit 1800 may receive commands related to operation control of the massage device 1000 from the user, and the user input unit 1800 may be implemented in various forms. For example, the user input unit 1800 may be provided in the body massage unit 100 or may be provided in the leg massage unit 300, but the present disclosure is not limited thereto.

The massage device 1000 may acquire various commands from the user through the user input unit 1800. For example, the massage device 1000 may receive an arbitrary command for selection of a massage module, selection of a massage type, selection of a massage intensity, selection of a massage period of time, selection of a massage part, selection of a position and operation of the body massage unit 100, selection of on/off of power of the massage device 1000, selection of whether to operate a heating function, or selection related to sound source playback, but the present disclosure is not limited thereto.

According to another embodiment of the present disclosure, the user input unit 1800 may include buttons in the form of a hot key and/or selection buttons for executing direction selection, cancellation, and input according to preset user setting functions or self-preset functions, etc.

The user input unit 1800 may be implemented as a keypad, a dome switch, a touch pad (static pressure/electrostatic), a jog wheel, a jog switch, and the like, but the present disclosure is not limited thereto. Further, the user input unit 1800 may acquire a command through the utterance of the user on the basis of voice recognition technology.

According to an embodiment of the present disclosure, the user input unit 1800 may include a display for displaying the operation state of the massage device 1000, the current state of the user, or the like. In this case, the display may include at least one of a liquid crystal display (LCD), a thin film transistor-liquid crystal display (TFT LCD), an organic light-emitting diode (OLED), a flexible display, and a three-dimensional (3D) display, but the present disclosure is not limited thereto.

The audio output unit 1600 may provide an arbitrary type of audio output to the user. For example, the audio output unit 1600 may provide brain stimulation to the user by outputting a sound source and/or binaural beats optimized for a massage pattern provided by the massage device 1000 to the user. The audio output unit 1600 may output an acoustic signal received through a network (not illustrated) or stored in an internal/external storage medium (not illustrated). For example, the audio output unit 1600 may be connected to a user terminal 2000 through a network (e.g., Bluetooth or the like) to output a sound source according to the control of the user terminal 2000. Further, the audio output unit 1600 may output an arbitrary type of acoustic signal generated in connection with the operation of the massage device 1000.

The massage device 1000 according to an embodiment of the present disclosure may include a network access unit 1400. The network access unit 1400 may communicate with the modules inside the massage device 1000, an external massage device, and/or the user terminal 2000 through an arbitrary type of network. The network access unit 1400 may include a wired/wireless access module for network access. As the wireless access technology, for example, a wireless local area network (WLAN) (i.e., Wi-Fi), a wireless broadband (WiBro), World Interoperability for Microwave Access (WiMAX), High Speed Downlink Packet Access (HSDPA), and the like may be used. As the wired access technology, for example, digital subscriber line (xDSL), Fiber to the Home (FTTH), power-line communication (PLC), and the like may be used. Further, the network access unit may include a short range communication module to transmit or receive data to or from an arbitrary device/terminal located at a short distance. For example, as the short range communication technology, Bluetooth, radio-frequency identification (RFID), Infrared Data Association (IrDA), ultra-wideband (UWB), ZigBee, and the like may be used as, but the present disclosure is not limited thereto.

A storage unit 1500 may store various pieces of information related to the massage device 1000. For example, the storage unit 1500 may include massage control information and include personal authentication information, but the present disclosure is not limited thereto.

The storage unit 1500 may be implemented using a non-volatile storage medium capable of continuously storing arbitrary data. For example, the storage unit 1500 may include disks, optical disks, and magneto-optical storage devices, and storage devices based on a flash memory and/or a battery-backed memory, but the present disclosure is not limited thereto.

Further, the storage unit 1500 may include a memory. The memory is a main storage device directly accessed by a processor, and may include a volatile storage device in which information stored therein is instantaneously erased when power is turned off, such as a random access memory (RAM) such as a dynamic random access memory (DRAM), a static random access memory (SRAM), or the like, but the present disclosure is not limited thereto. Such a memory may be operated by the control unit 1200.

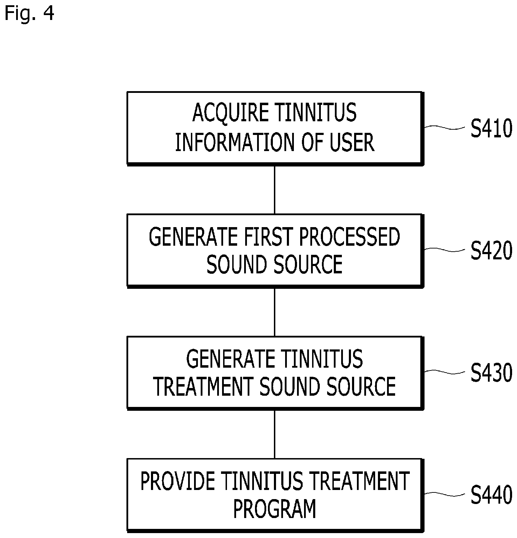

FIG. 4 is a flowchart for describing a method by which a massage device provides a tinnitus treatment program according to an embodiment of the present disclosure.

In S410, the massage device 1000 may acquire tinnitus information of a user. For example, the massage device 1000 may acquire a tinnitus frequency band of the user.

Frequencies at which tinnitus occurs for each user may be different from each other, and the tinnitus frequency band refers to a frequency band in which tinnitus mainly occurs for users.

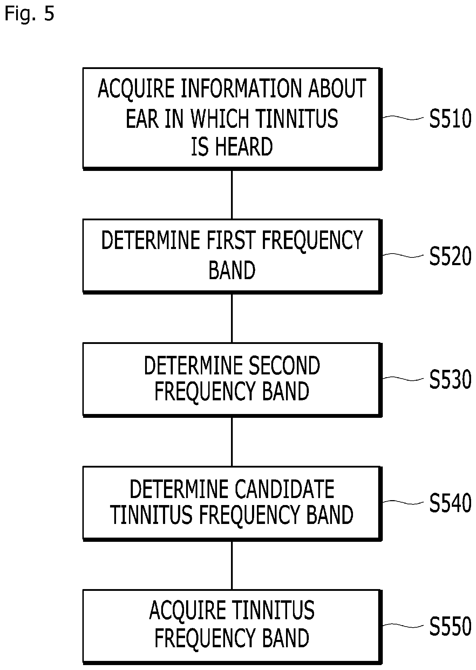

The massage device 1000 may acquire a tinnitus frequency band of the user in various ways. For example, the massage device 1000 may directly determine the tinnitus frequency band of the user or may acquire the tinnitus frequency band of the user from an external device. Hereinafter, a method of acquiring a tinnitus frequency band according to an embodiment of the present disclosure will be described with reference to FIG. 5.

FIG. 5 is a flowchart for describing a method of acquiring a tinnitus frequency band according to an embodiment of the present disclosure.

In S510, the massage device 1000 may acquire information about an ear in which tinnitus is heard. For example, the massage device 1000 may provide an interface in which the user can select a left ear, a right ear, or both ears to the user. The user may select the ear in which tinnitus occurs through the provided user interface, and the massage device 1000 may acquire the information about the ear in which tinnitus occurs on the basis of information about the selection by the user.

Further, the massage device 1000 may acquire the information about the ear in which tinnitus occurs, which is provided from an external device to the user in advance, but the present disclosure is not limited thereto.

In S520, the massage device 1000 may determine a first frequency band in which tinnitus occurs.

For example, the massage device 1000 may output a sound source to the left ear, the right ear, or both ears on the basis of the information about the ear in which tinnitus occurs, and may provide an interface in which the user can select a frequency band in which tinnitus occurs together with the sound source.

Specifically, the massage device 1000 may output a sound source whose frequency is gradually increased from a low frequency band to a high frequency band (e.g., a sound source whose frequency is gradually increased from 250 Hz to 8,000 Hz for 1 minute is output), provide the interface in which the user can select a frequency band in which tinnitus occurs to the user, and allow the user to directly select the frequency band in which tinnitus occurs.

In S530, the massage device 1000 may determine a second frequency band in which tinnitus occurs.

For example, the massage device 1000 may output a sound source to the left ear, the right ear, or both ears on the basis of the information about the ear in which tinnitus occurs, and may provide an interface in which the user can select a frequency band in which tinnitus occurs together with the sound source.

Specifically, the massage device 1000 may output a sound source whose frequency is gradually reduced from a high frequency band to a low frequency band (e.g., a sound source whose frequency is gradually reduced from 8,000 Hz to 250 Hz for 1 minute is output), provide the interface in which the user can select a frequency band in which tinnitus occurs to the user, and allow the user to directly select the frequency band in which tinnitus occurs.

In S540, the massage device 1000 may determine a candidate tinnitus frequency band.

For example, the massage device 1000 may determine the candidate tinnitus frequency band on the basis of the determined first and the second frequency bands.

Specifically, the massage device 1000 may select at least one frequency band close to the first and second frequency bands from among predetermined frequency bands (e.g., 250 Hz, 500 Hz, 1,000 Hz, 2,000 Hz, 3,000 Hz, 4,000 Hz, 6,000 Hz, and 8,000 Hz) and determine the selected frequency band as the candidate tinnitus frequency band.

In S550, the massage device 1000 may acquire a tinnitus frequency band. For example, the massage device 1000 may provide the candidate tinnitus frequency band to the user through the interface, and acquire the tinnitus frequency band of the user on the basis of the information about the selection by the user through the interface.

In S420, the massage device 1000 may generate a first processed sound source.

According to an embodiment of the present disclosure, the massage device 1000 may generate a first processed sound source by adjusting the magnitude of at least some frequency components among a plurality of frequency components included in a raw sound source and flattening the raw sound source.

The raw sound source refers to a part of an entire sound source that does not include a human voice.

The entire sound source may include a voice-containing part that includes the human voice, a voice-excluded part that does not include the human voice, or a combination thereof. The voice-containing part may include a part for guiding the user's action with the human voice, a part for singing with the human voice, and the like, and the voice-excluded part may include an accompaniment part.

The massage device 1000 may determine a raw sound source by excluding a voice-containing part that includes the human voice from the entire sound source.

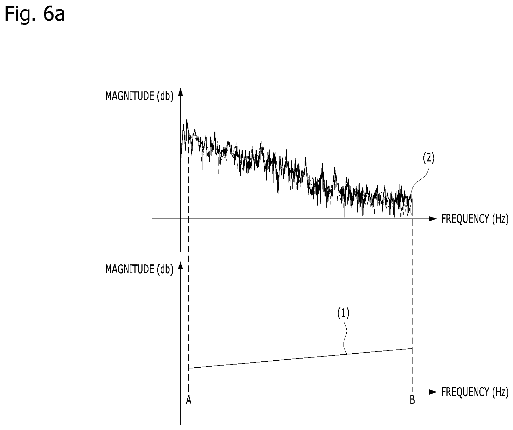

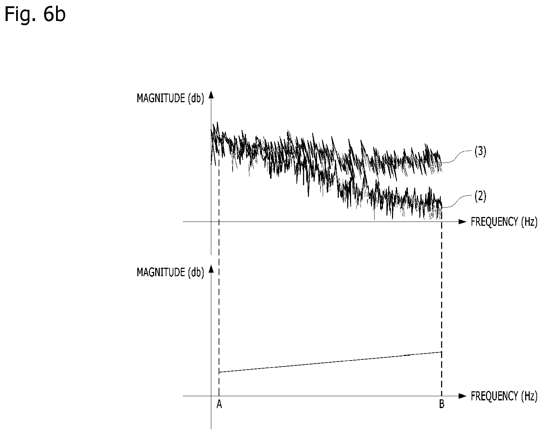

For example, the massage device 1000 may generate the first processed sound source by adjusting the magnitude of at least some frequency components among the plurality of frequency components included in the raw sound source on the basis of a virtual line having a slope. This will be described in detail with reference to FIGS. 6A and 6B.

FIGS. 6A and 6B are graphs for describing a method of generating a first processed sound source according to an embodiment of the present disclosure.

According to an embodiment of the present disclosure, the massage device 1000 may generate a first processed sound source by adjusting the magnitude of at least some frequency components among a plurality of frequency components included in a raw sound source on the basis of a virtual line having a slope.

A virtual line (1) of FIG. 6A shows a virtual line having a slope, and a line (2) of FIG. 6A shows a raw sound source on a plane having a frequency component (Hz) and a magnitude (dB).

The massage device 1000 may flatten the raw sound source by adjusting the magnitude (dB) of at least some sections (e.g., a section between A and B) among all sections of the raw sound source according to the virtual line (1) having a slope.

FIG. 6B is a view showing a line (3) indicating a first processed sound source which is generated by flattening the raw sound source and a line (2) indicating a raw sound source.

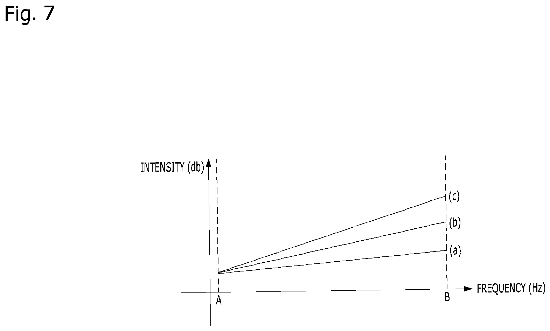

In this case, the slope of the virtual line may be variously determined. This will be described with reference to FIG. 7.

FIG. 7 is a graph for describing a method of determining a slope of a virtual line according to an embodiment of the present disclosure.

According to an embodiment of the present disclosure, the massage device 1000 may generate a first processed sound source by flattening a raw sound source on the basis of a predetermined slope. In this case, the slope may be applied differently according to the user.

The massage device 1000 may determine the slope on the basis of a sympathetic hyperactivity level of the user.

For example, the massage device 1000 may determine the sympathetic hyperactivity level of the user, and determine the slope on the basis of the determined sympathetic hyperactivity level. Specifically, the massage device 1000 may acquire HRV information of the user, determine the sympathetic hyperactivity level of the user by utilizing a ratio (LF/HF ratio) of power in a low frequency band to power in a high frequency band included in the HRV information, and determine the slope on the basis of the determined sympathetic hyperactivity level.

When excessive stimulation is applied to the user whose sympathetic nerve is hyperactive, the user may feel uncomfortable. Accordingly, the massage device 1000 may generate a sound source with less stimulation by generating the first processed sound source on the basis of a line (c) having a steep slope for a user whose sympathetic nerve is relatively hyperactive.

According to another embodiment of the present disclosure, a virtual line may be implemented as a curve. Further, the virtual line may be implemented as a stepped line, but the present disclosure is not limited thereto.

When the virtual line is implemented as a curve, a slope of the virtual line may be replaced with a slope of the curve. Further, when the virtual line is implemented as a stepped line, a slope of the virtual line may be replaced with a slope of a straight line that connects a start point and an end point of the stepped line, but the present disclosure is not limited thereto.

In S430, the massage device 1000 may generate a tinnitus treatment sound source.

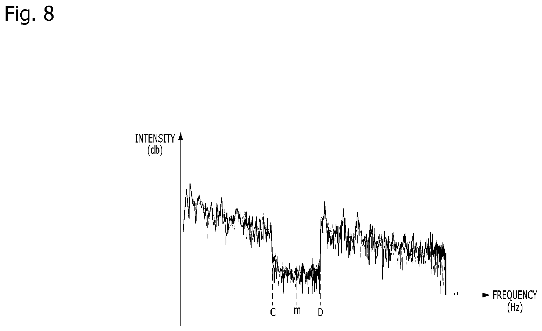

According to an embodiment of the present disclosure, the massage device 1000 may generate the tinnitus treatment sound source by reducing the magnitude of at least some frequency components among a plurality of frequency components included in the first processed sound source on the basis of tinnitus information. This will be described with reference to FIG. 8.

FIG. 8 is a graph for describing a method of generating a tinnitus treatment sound source according to an embodiment of the present disclosure.

In FIG. 8, m denotes a tinnitus frequency band of the user. The massage device 1000 may reduce a magnitude of a frequency component by as much as a predetermined band range (e.g., by as much as a section between C and D) based on the tinnitus frequency band of the user. In this case, C may represent m/√2, and D may represent m^{∗}√2 (here, √2 is an irrational number that becomes 2 when squared), but the present disclosure is not limited thereto.

The massage device 1000 may generate a tinnitus treatment sound source by utilizing a notch filter, but the present disclosure is not limited thereto.

In S440, the massage device 1000 may provide a tinnitus treatment program in which a tinnitus treatment sound source and a tinnitus treatment massage are combined.

According to an embodiment of the present disclosure, the massage device 1000 may generate the tinnitus treatment program by combining the tinnitus treatment sound source and the tinnitus treatment massage, and provide the generated tinnitus treatment program to the user.

According to an embodiment of the present disclosure, the tinnitus treatment massage may be determined adaptively according to the user. For example, the massage device 1000 may determine the tinnitus treatment massage on the basis of the sympathetic hyperactivity level. Specifically, the massage device 1000 may acquire HRV information, determine the sympathetic hyperactivity level of the user by utilizing a ratio (LF/HF ratio) of power in a low frequency band to power in a high frequency band included in the HRV information, and determine the tinnitus treatment massage on the basis of the sympathetic hyperactivity level.

When excessive stimulation is applied to the user whose sympathetic nerve is hyperactive, the user may feel uncomfortable, and accordingly, the effectiveness of the tinnitus treatment program may be reduced. In order to solve the above problem, the massage device 1000 may provide a gentle massage (e.g., a massage performed with relatively weak kneading and tapping, a massage performed with a massage module relatively less protruding, and/or a massage performed evenly throughout, rather than intensively massaging one part) to the user whose sympathetic nerve is relatively hyperactive.

According to another embodiment of the present disclosure, the tinnitus treatment massage may be determined based on biometric information of the user. For example, the massage device 1000 may measure a stress index by analyzing the HRV information of the user. In this case, when it is determined that the stress index is high, the massage device 1000 may determine a relatively strong upper body massage as the tinnitus treatment massage. Further, when it is determined that the stress index is low, the massage device 1000 may determine an overall gentle massage (e.g., a massage performed with relatively weak kneading and tapping, a massage performed with a massage module relatively less protruding, and/or a massage performed evenly throughout, rather than intensively massaging one part) as the tinnitus treatment massage.

When the user is provided with the tinnitus treatment program in the massage device according to an embodiment of the present disclosure, habituation of perception of tinnitus may be induced.

For example, the massage device 1000 may relieve an unpleasant autonomic symptom and transmit a patient-specific sound source that enables effective stimulation by using the lateral inhibition phenomenon of neurons in the tinnitus frequency band, and thus habituation of perception of tinnitus may be induced.

Such a sound source may be applied to tinnitus patients by making the magnitude of each frequency constant through a fast Fourier transform. Further, the corresponding sound source may induce habituation of reaction of tinnitus, including a tinnitus retraining treatment program produced by experts such as otolaryngologists and psychiatrists, and a massage device 1000 that can treat the pathophysiology of tinnitus as a whole may be provided through the classical conditioning of parasympathetic nerve stimulation of the massage chair and tinnitus frequency band nerve cell stimulation.

It will be appreciated by those skilled in the art that the present disclosure may be implemented in combination with other program modules and/or as a combination of hardware and software. For example, the present disclosure may be embodied by a computer-readable medium.

Any medium accessible by a computer may be a computer-readable medium, and such computer-readable media include volatile and non-volatile media, transitory and non-transitory media, removable and non-removable media. As a non-limiting example, the computer-readable media may include computer-readable storage media and computer-readable transmission media.

The computer-readable storage media include volatile and non-volatile media, transitory and non-transitory media, and removable and non-removable media which are implemented in any method or technology for storing information such as computer readable instructions, data structures, program modules, or other data. The computer-readable storage media include an RAM, a read only memory (ROM), an electrically erasable programmable ROM (EEPROM), a flash memory or other memory technology, a compact disc read only memory (CD-ROM), a digital video disc (DVD) or other optical disk storage devices, a magnetic cassette, magnetic tape, a magnetic disk storage device, or other magnetic storage devices, or any other medium that can be accessed by a computer and used to store the desired information, but the present disclosure is not limited thereto.

The computer-readable transmission media typically embody computer readable instructions, data structures, program modules, or other data in a modulated data signal, such as a carrier wave or other transport mechanisms, and include any information delivery medium. The term "modulated data signal" refers to a signal in which one or more of the characteristics of the signal is set or changed so as to encode information in the signal. As a non-limiting example, the computer-readable transmission media include wired media such as a wired network or direct-wired connection, and wireless media such as acoustic, radio frequency (RF), infrared, and other wireless media. Combinations of any media described above are also intended to be included within the scope of computer-readable transmission media.

It will be understood by those skilled in the art that various illustrative logical blocks, modules, processors, means, circuits, and algorithm steps described in connection with the embodiments disclosed herein may be implemented by electronic hardware, various forms of program or design code (referred to herein as "software" for convenience of description), or a combination thereof. In order to clearly describe such interchangeability of hardware and software, various illustrative components, blocks, modules, circuits, and steps have been described above generally in terms of their functionality. Whether such functionality is implemented as hardware or software depends upon the specific application and design constraints imposed on the overall system. Those skilled in the art may implement the described functionality in various ways for each specific application, but such implementation decisions should not be interpreted as departing from the scope of the present disclosure.

The various embodiments presented herein may be implemented as methods, devices, or articles of manufacture using standard programming and/or engineering techniques. The term "article of manufacture" includes a computer program, carrier, or media accessible from any computer-readable device. For example, the computer-readable storage media include magnetic storage devices (e.g., hard disks, floppy disks, magnetic strips, etc.), optical disks (e.g., CDs, DVDs, etc.), smart cards, and flash memory devices (e.g., EEPROMs, cards, sticks, key drives, etc.), but the present disclosure is not limited thereto. The term "machine-readable media" include wireless channels and various other media capable of storing, retaining, and/or carrying instruction(s) and/or data, but the present disclosure is not limited thereto.

It is understood that the specific order or hierarchy of steps in the presented processes is an example of exemplary approaches. Based on design priorities, it should be understood that the specific order or hierarchy of steps in the processes may be rearranged within the scope of the present disclosure. The appended method claims present elements of the various steps in a sample order, but are not meant to be limited to the specific order or hierarchy presented.

The description of the presented embodiments is provided to enable those skilled in the art to make or use the present disclosure. Various modifications to these embodiments will be readily apparent to those skilled in the art, and the generic principles defined herein may be applied to other embodiments without departing from the scope of the present disclosure. Therefore, the present disclosure is not to be limited to the embodiments presented herein, but is to be construed in the widest scope consistent with the principles and novel features presented herein.

## Claims

1. A method by which a massage device provides a tinnitus treatment program, comprising:
acquiring tinnitus information of a user;
generating a first processed sound source by adjusting a magnitude of at least some frequency components among a plurality of frequency components included in a raw sound source and flattening the raw sound source;
generating a tinnitus treatment sound source by reducing a magnitude of at least some frequency components among a plurality of frequency components included in the first processed sound source on the basis of the acquired tinnitus information; and
providing a tinnitus treatment program in which the generated tinnitus treatment sound source and a tinnitus treatment massage are combined.

2. The method of claim of 1, wherein, in the generating of the first processed sound source, the first processed sound source is generated by adjusting the magnitude of at least some frequency components among the plurality of frequency components included in the raw sound source on the basis of a virtual line having a slope.

3. The method of claim of 1, wherein the slope is determined based on a sympathetic hyperactivity level of the user.

4. The method of claim of 3, wherein the massage device acquires heart rate variability (HRV) information of the user and determines the sympathetic hyperactivity level of the user by utilizing a ratio (LF/HF ratio) of power in a low frequency band to power in a high frequency band included in the HRV information.

5. The method of claim of 1, further comprising determining the raw sound source from an entire sound source,
wherein, in the determining of the raw sound source from the entire sound source, the raw sound source is determined by excluding a part that includes a human voice from the entire sound source.

6. The method of claim of 1, wherein heart rate variability (HRV) information of the user is acquired, and the tinnitus treatment massage is determined by utilizing a ratio (LF/HF ratio) of power in a low frequency band to power in a high frequency band included in the HRV information.

7. The method of claim of 1, wherein the acquiring of the tinnitus information includes:
providing a user interface to select an ear in which tinnitus is heard, and acquiring information about the ear in which tinnitus is heard through the provided user interface;
outputting a sound source whose frequency is gradually increased from a low frequency, and determining a first frequency band in which tinnitus is heard on the basis of selection information received from the user;
outputting a sound source whose frequency is gradually reduced from a high frequency, and determining a second frequency band in which tinnitus is heard on the basis of the selection information received from the user;
determining at least one candidate tinnitus frequency band on the basis of the first and second frequency bands; and
outputting a sound source matching the candidate tinnitus frequency band, and acquiring a tinnitus frequency band on the basis of the selection information received from the user.

8. A massage device providing a tinnitus treatment program, comprising:
a tinnitus information acquisition unit configured to acquire tinnitus information of a user;
a first processed sound source generation unit configured to generate a first processed sound source by adjusting a magnitude of at least some frequency components among a plurality of frequency components included in a raw sound source and flattening the raw sound source;
a tinnitus treatment sound source generation unit configured to generate a tinnitus treatment sound source by reducing a magnitude of at least some frequency components among a plurality of frequency components included in the first processed sound source on the basis of the acquired tinnitus information; and
a tinnitus treatment program providing unit configured to provide a tinnitus treatment program in which the generated tinnitus treatment sound source and a tinnitus treatment massage are combined.
